# EUROPEAN PATENT APPLICATION

(11) **EP 4 311 569 A1**
(43) Date of publication of application: **31.01.2024**
(21) Application number: 23188203.6
(22) Date of filing: 27.07.2023
(51) Int. Cl.: A61M 39/04, A61M 39/22

(54) **HYDRAULICALLY ACTUATED SUBCUTANEOUS VALVE DEVICE, SYSTEM AND METHOD**

(30) Priority: 29.07.2022 US 202263393500 P; 29.06.2023 US 202318344768
(71) Applicant: Medtronic MiniMed, Inc., Northridge, CA 91325-1219 (US)
(72) Inventor: Smith, Rodger E., Ivins, 84087 (US)
(74) Representative: Zimmermann & Partner Patentanwälte mbB

(57) **Abstract**

A hydraulically actuated valve device includes a valve body having an inner cavity, an inlet channel extending to the inner cavity, an outlet channel extending from the inner cavity, and an open end that opens to the inner cavity. A septum is located in the inner cavity to seal the open end of the valve body and to receive a needle through which an operating fluid may be selectively injected into or withdrawn from the inner cavity to increase or reduce a fluid pressure in the inner cavity. A valve closing member has an unactuated state to enable a first fluid to flow from the inlet channel to the outlet channel when the fluid pressure of operating fluid in the inner cavity of the valve body is below a closing pressure, and inhibits the first fluid from flowing out of the inner cavity through the outlet channel when the operating fluid in the inner cavity of the valve body is at or above the closing pressure.

## Description

### CROSS-REFERENCE TO RELATED APPLICATION

This application claims the benefit of U.S. Patent Application No. 63/393,500, filed July 29, 2022, entitled "HYDRAULICALLY ACTUATED SUBCUTANEOUS VALVE DEVICE, SYSTEM AND METHOD," the full disclosure of which is incorporated herein by reference in its entirety.

### BACKGROUND

The present disclosure relates to hydraulically actuated subcutaneous valves devices and methods of making and using the same, and to medical devices, systems or methods that include or operate with such valve devices. In some examples, such medical devices, systems or methods include subcutaneously implantable infusion pumps, subcutaneous sensors or other subcutaneously implantable medical devices and systems having at least one fluid flow conduit through which a fluid flow may be selectively controlled or stopped by one or more hydraulically actuated valve devices as described herein.

Certain biological conditions may be treated, monitored or sensed, according to modern medical techniques that include or employ one or more subcutaneously implantable medical devices. Various types of such medical devices have one or more fluid conduits through which a drug, treatment fluid, marker, biological fluid or other fluid is conveyed to or from a port (e.g., an outlet port or an inlet port) during operation of the device. For example, implantable infusion pumps typically include one or more fluid flow conduits through which an infusion fluid may be selectively conveyed to and through an outlet port. The outlet port may be connected to one or more implantable cannulas, for conveying the infusion fluid from the outlet port to a selected location in a patient's body. Similarly, implantable sensor or monitor devices may include one or more fluid flow conduits through which a biological fluid may be conveyed to or from a sensor element.

In some contexts of use, a subcutaneously implanted medical device may require or benefit from a reliable cleaning or flushing of the fluid conduits and other fluid-contacting components of the device, for example, after a particular period of operation. In some cases, a periodic cleaning or flushing program is desirable. However, it is typically desirable to avoid introducing cleaning or flushing fluid into the patient's body, from the implanted device. In certain cases, implanted medical devices have been surgically removed from the patient for a cleaning or flushing procedure, and then surgically re-implanted in the patient, after cleaning or flushing.

### SUMMARY

Examples described herein relate to hydraulically actuated subcutaneous valves and implantable devices and systems that include such valves. Further examples relate to methods of making and using such devices and systems. In particular examples, the hydraulically actuated subcutaneous valves are configured to be selectively actuated to close or to open, through a transcutaneous-inserted syringe or other skin-piercing device.

In certain examples, an implanted, hydraulically actuated subcutaneous valve is actuated, transcutaneously, to be in a closed state, to close a fluid conduit and fluidically isolate any fluid within the device from the patient. While the valve is in the closed state, a cleaning or flushing fluid media is transcutaneously communicated to the implanted medical device, for example, from a syringe or other transcutaneous fluid delivery device. The closed valve prevents or inhibits the cleaning or flushing fluid from flowing out from a port on the medical device to the patient. The cleaning or flushing media may be withdrawn from the implanted medical device, for example, by a syringe or other transcutaneous fluid receiving device. Thereafter, the hydraulically actuated subcutaneous valve may be returned to an open state (or de-actuated), to enable fluid flow through the fluid conduit of the medical device during subsequent operation of the medical device. In particular examples, the hydraulically actuated subcutaneous valve may be selectively actuated and de-actuated, transcutaneously, without requiring a surgical removal and replacement of the valve device or of the medical device containing the valve device.

A hydraulically actuated valve device according to certain examples described herein includes a valve body having an inner cavity, an inlet channel extending to the inner cavity, an outlet channel extending from the inner cavity, and an open end that opens to the inner cavity. A septum is located in the inner cavity, is arranged to seal the open end of the valve body and is configured to receive a needle through which an operating fluid may be selectively injected into the inner cavity of the valve body to increase a fluid pressure in the inner cavity of the valve body, or selectively withdrawn from the inner cavity to reduce the fluid pressure in the inner cavity. A valve closing member is arranged in the inner cavity of the valve body. The valve closing member has an unactuated state to enable a first fluid to flow from the inlet channel to the outlet channel when the fluid pressure of operating fluid in the inner cavity of the valve body is below a closing pressure. The valve closing member has an actuated state that inhibits the first fluid from flowing into the inner cavity from the inlet channel or out of the inner cavity through the outlet channel when the operating fluid in the inner cavity of the valve body is at or above the closing pressure.

In a hydraulically actuated valve device according to further examples, the valve closing member comprises a moveable member that is configured to be in a first position within the inner cavity of the valve body when the fluid pressure of operating fluid in the inner cavity of the valve body is below the closing pressure, and to move to a second position within the inner cavity of the valve body when the fluid pressure of the operating fluid in the inner cavity of the valve body is at or above the closing pressure. The moveable member enables the first fluid to flow from the inlet channel to the outlet channel when the moveable member is in the first position. The moveable member obstructs at least one of the inlet channel and the outlet channel to inhibit flow of the first fluid from the inlet channel to the outlet channel when the moveable member is in the second position.

In a hydraulically actuated valve device according to further examples, the moveable member includes at least one seal member that provides a fluid seal between the moveable member and an inner wall of the cavity of the valve body.

In a hydraulically actuated valve device according to further examples, the at least one seal includes at least one O-ring disposed around the moveable member.

In a hydraulically actuated valve device according to further examples, the valve closing member includes a diaphragm located within the cavity of the valve body.

In a hydraulically actuated valve device according to further examples, the diaphragm is a deformable diaphragm configured to be in a first state when the fluid pressure of operating fluid in the inner cavity of the valve body is below the closing pressure, and to deform from the first state to a second state when the fluid pressure of the operating fluid in the inner cavity of the valve body is at or above the closing pressure. The deformable diaphragm enables the first fluid to flow from the inlet channel to the outlet channel when the deformable diaphragm is in the first state. The deformable diaphragm obstructs at least one of the inlet channel and the outlet channel to inhibit flow of the first fluid from the inlet channel to the outlet channel when the deformable diaphragm is in the second state.

In a hydraulically actuated valve device according to further examples, the valve body has a valve seat in a flow path between the inlet channel and the outlet channel, and the diaphragm is configured to enable flow of the first fluid from the inlet channel, through the flow path, to the outlet channel when the fluid pressure of the operating fluid in the inner cavity of the valve body is below the closing pressure, and to seal against the valve seat and inhibit flow of the first fluid to the outlet channel when the fluid pressure of the operating fluid in the inner cavity of the valve body is at or above the closing pressure.

In a hydraulically actuated valve device according to further examples, the diaphragm is a deformable diaphragm configured to deform from a first state to a second state as the fluid pressure of the operating fluid in the inner cavity of the valve body is raised from below to above the closing pressure.

A hydraulically actuated valve device according to further examples, further includes a flexible tubing in the inner cavity of the valve body and extending between the inlet channel and the outlet channel, for flow of the first fluid from the inlet channel to the outlet channel when the fluid pressure of the operating fluid in the inner cavity of the valve body is below the closing pressure. The diaphragm has a protruding obstruction feature configured to press against and close the flexible tubing to inhibit flow of the first fluid from the inlet channel to the outlet channel when the fluid pressure of the operating fluid in the inner cavity of the valve body is at or above the closing pressure.

A hydraulically actuated valve device according to further examples, further includes a needle guard comprising a rigid body resistant to being pierced by the needle. The rigid body of the needle guard is located in the inner cavity of the valve body between the septum and the diaphragm, to protect the diaphragm from needle piercing.

In a hydraulically actuated valve device according to further examples, the rigid body of the needle guard includes at least one fluid flow passage through which the operating fluid may flow when the operating fluid is injected into the inner cavity of the valve body.

In a hydraulically actuated valve device according to further examples, the rigid body of the needle guard has an outer peripheral edge that includes a plurality channels defining fluid flow passages through which the operating fluid may flow when the operating fluid is injected into the inner cavity of the valve body.

In a hydraulically actuated valve device according to further examples, the valve closing member includes a flexible tubing in the inner cavity of the valve body and extending between the inlet channel and the outlet channel, for flow of the first fluid from the inlet channel to the outlet channel when the fluid pressure of the operating fluid in the inner cavity of the valve body is below the closing pressure. The flexible tubing configured to collapse under fluid pressure of the operating fluid in the inner cavity of the valve body and inhibit flow of the first fluid from the inlet channel to the outlet channel when the fluid pressure of the operating fluid in the inner cavity of the valve body is at or above the closing pressure.

A hydraulically actuated valve device according to further examples, further includes a needle guard having a rigid body resistant to being pierced by the needle. The rigid body of the needle guard is located in the inner cavity of the valve body between the septum and the flexible tubing of the valve closing member, to protect the flexible tubing from needle piercing.

In a hydraulically actuated valve device according to further examples, the rigid body of the needle guard includes at least one fluid flow passage through which the operating fluid may flow when the operating fluid is injected into the inner cavity of the valve body.

A hydraulically actuated valve device according to further examples, further includes a needle guide located on the open end of the valve body for guiding the needle toward a central portion of the septum.

In a hydraulically actuated valve device according to further examples, the needle guide has an annular body having a tapered surface extending to a central opening through which the needle may be passed.

Further examples relate to an implantable medical device having a hydraulically actuated valve device of any one of the above examples, and a fluid outlet port connected to the outlet channel of the valve body.

Further examples relate to an implantable medical device having a hydraulically actuated valve device of any one of the above examples, and a fluid inlet port connected to the inlet channel of the valve body.

Further examples relate to an implantable medical device having an outlet port, an infusion media pump for selectively delivering the first fluid to the outlet port, and a hydraulically actuated valve device of any one of the above examples connected between the infusion media pump and the outlet port.

Further disclosed herein is a hydraulically actuated valve device that includes a valve body having an inner cavity, an inlet channel extending to the inner cavity, an outlet channel extending from the inner cavity, and an open end that opens to the inner cavity. A septum is located in the inner cavity to seal the open end of the valve body and to receive a needle through which an operating fluid may be selectively injected into or withdrawn from the inner cavity to increase or reduce a fluid pressure in the inner cavity. A valve closing member has an unactuated state to enable a first fluid to flow from the inlet channel to the outlet channel when the fluid pressure of operating fluid in the inner cavity of the valve body is below a closing pressure, and inhibits the first fluid from flowing out of the inner cavity through the outlet channel when the operating fluid in the inner cavity of the valve body is at or above the closing pressure.

### BRIEF DESCRIPTION OF THE DRAWINGS

The above and other aspects and features of the present invention will become more apparent to those skilled in the art from the following detailed description of the example embodiments with reference to the accompanying drawings, in which:
FIG. 1 is a perspective view of an example of a hydraulically actuated subcutaneous valve device.
FIG. 2 is an exploded, perspective view of the example of the hydraulically actuated subcutaneous valve device of FIG. 1.
FIG. 3 is a cross-section view of the example of the hydraulically actuated subcutaneous valve device of FIG. 1 in an unactuated (or open) state.
FIG. 4 is another cross-section view of the example of the hydraulically actuated subcutaneous valve device of FIG. 1 in an actuated (or closed) state.
FIG. 5 is a cross-section view of the example of the hydraulically actuated subcutaneous valve device of FIG. 1 with a syringe needle inserted therein.
FIG. 6 is an enlarged cross-section view of a portion of the hydraulically actuated subcutaneous valve device in FIG. 5.
FIG. 7 is a cross-section view of another example of a hydraulically actuated subcutaneous valve device in an unactuated (or open) state.
FIG. 8 is a cross-section view of the example of the hydraulically actuated subcutaneous valve device of FIG. 7, in an actuated (or closed) state.
FIG. 9 is a perspective view of an example of a needle guard.
FIG. 10 is a cross-section view of another example of a hydraulically actuated subcutaneous valve device in an unactuated (or open) state.
FIG. 11 is a cross-section view of the example of the hydraulically actuated subcutaneous valve device of FIG. 10, in an actuated (or closed) state.
FIG. 12 is a perspective view of an example of a hydraulically deformable membrane.
FIG. 13 is a cross-section view of another example of a hydraulically actuated subcutaneous valve device in an unactuated (or open) state.
FIG. 14 is a cross-section view of the example of the hydraulically actuated subcutaneous valve device of FIG. 13, in an actuated (or closed) state.
FIG. 15 is a perspective view of an example of a medical device having a hydraulically actuated subcutaneous valve device.
FIG. 16 is a schematic diagram of the medical device of FIG. 15.
FIG. 17 is a perspective view of an example of a system that includes a medical device and a hydraulically actuated subcutaneous valve device.
FIG. 18 is a generalized schematic diagram of a medical device or system having a hydraulically actuated subcutaneous valve device, implanted in a patient.

### DETAILED DESCRIPTION

Hereinafter, example embodiments will be described in more detail with reference to the accompanying drawings. The present invention, however, may be embodied in various different forms, and should not be construed as being limited to only the illustrated embodiments herein. Rather, these embodiments are provided as examples so that this disclosure will be thorough and complete, and will fully convey the aspects and features of the present invention to those skilled in the art. Accordingly, processes, elements, and techniques that are not necessary to those having ordinary skill in the art for a complete understanding of the aspects and features of the present invention may not be described. Unless otherwise noted, like reference numerals denote like elements throughout the attached drawings and the written description, and thus, descriptions thereof may not be repeated. Further, features or aspects within each example embodiment should typically be considered as available for other similar features or aspects in other example embodiments.

Certain terminology may be used in the following description for the purpose of reference only, and thus are not intended to be limiting. For example, terms such as "top", "bottom", "upper", "lower", "above", and "below" could be used to refer to directions in the drawings to which reference is made. Terms such as "front", "back", "rear", "side", "outboard", and "inboard" could be used to describe the orientation and/or location of portions of the component within a consistent but arbitrary frame of reference which is made clear by reference to the text and the associated drawings describing the component under discussion. Such terminology may include the words specifically mentioned above, derivatives thereof, and words of similar import. Similarly, the terms "first", "second", and other such numerical terms referring to structures do not imply a sequence or order unless clearly indicated by the context.

It will be understood that when an element or feature is referred to as being "on," "secured to," "connected to," or "coupled to" another element or layer, it can be directly on, connected to, or coupled to the other element or feature, or one or more intervening elements or features may be present. In addition, it will also be understood that when an element or features is referred to as being "between" two elements or features, it can be the only element or feature between the two elements or features, or one or more intervening elements or features may also be present.

The terminology used herein is for the purpose of describing particular embodiments and is not intended to be limiting of the present invention. As used herein, the singular forms "a" and "an" are intended to include the plural forms as well, unless the context clearly indicates otherwise. It will be further understood that the terms "comprises," "comprising," "includes," and "including," "has, " "have, " and "having," when used in this specification, specify the presence of the stated features, integers, steps, operations, elements, and/or components, but do not preclude the presence or addition of one or more other features, integers, steps, operations, elements, components, and/or groups thereof. As used herein, the term "and/or" includes any and all combinations of one or more of the associated listed items. Expressions such as "at least one of," when preceding a list of elements, modify the entire list of elements and do not modify the individual elements of the list.

As used herein, the term "substantially," "about," and similar terms are used as terms of approximation and not as terms of degree, and are intended to account for the inherent variations in measured or calculated values that would be recognized by those of ordinary skill in the art. Further, the use of "may" when describing embodiments of the present invention refers to "one or more embodiments of the present invention." As used herein, the terms "use," "using," and "used" may be considered synonymous with the terms "utilize," "utilizing," and "utilized," respectively.

Unless otherwise defined, all terms (including technical and scientific terms) used herein have the same meaning as commonly understood by one of ordinary skill in the art to which the present invention belongs. It will be further understood that terms, such as those defined in commonly used dictionaries, should be interpreted as having a meaning that is consistent with their meaning in the context of the relevant art and/or the present specification, and should not be interpreted in an idealized or overly formal sense, unless expressly so defined herein.

As described above, example embodiments relate to hydraulically actuated subcutaneous valve devices and methods of making and using the same. Further example embodiments relate to various medical devices, systems or methods that include or operate with such hydraulically actuated subcutaneous valve devices. Certain examples of the hydraulically actuated subcutaneous valve devices described herein are configured to be implanted (or are included in a medical device that is configured to be implanted) in a patient. In those examples, the hydraulically actuated subcutaneous valve devices may be selectively actuated or de-actuated, in a transcutaneous manner, with a needle inserted through the patient's skin. In some examples, the implant site may be in the abdomen of the patient. In other examples, the implant site may be located in other suitable implant site locations of a patient.

In particular examples, one or more of the hydraulically actuated subcutaneous valve devices described herein is/are in an implantable medical device or system that includes or operates with one or more fluid flow conduits through which a first fluid, such as, but not limited to a drug, treatment fluid, marker, biological fluid or other infusion media or fluid is conveyed to or from a port (e.g., an outlet port or an inlet port of the medical device or system) during operation of the medical device or system. The drug, treatment fluid, marker, biological fluid or other fluid that is conveyed through the one or more fluid flow conduits of the implantable medical device or system is referred to herein as a first fluid, to distinguish it from a valve operating fluid (or a second fluid) used to selectively actuate the hydraulically actuated subcutaneous valve devices described herein, and from a flushing fluid used to selectively clean or flush a medical device, as described herein.

In some examples, the medical device or system is or includes an implantable infusion pump configured to selectively dispense an infusion fluid to the patient, through one or more outlet ports. In other examples, the medical device is or includes a sensor for sensing one or more analytes or biological conditions from a biological fluid received from the patient through one or more inlet ports. In yet other examples, the hydraulically actuated subcutaneous valve devices described herein are included in other types of medical devices.

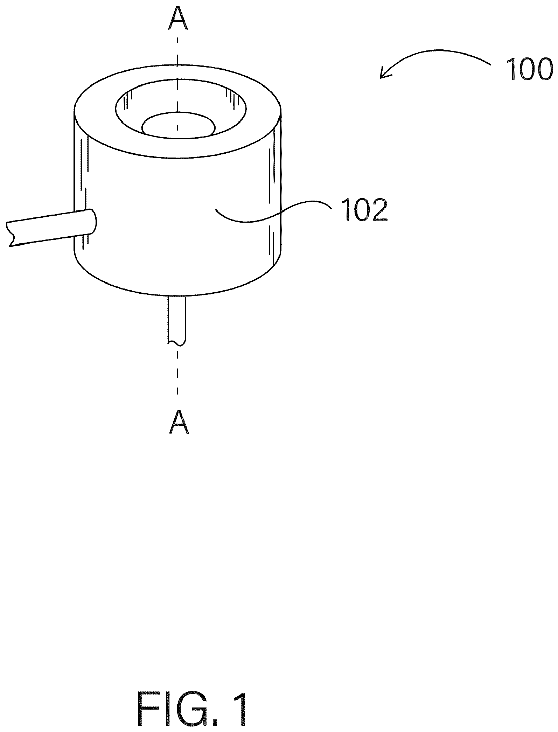

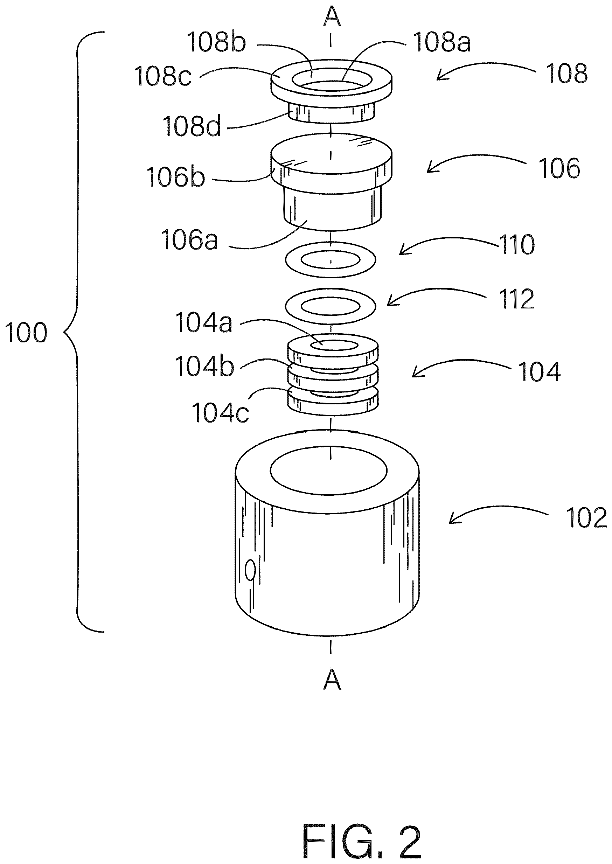

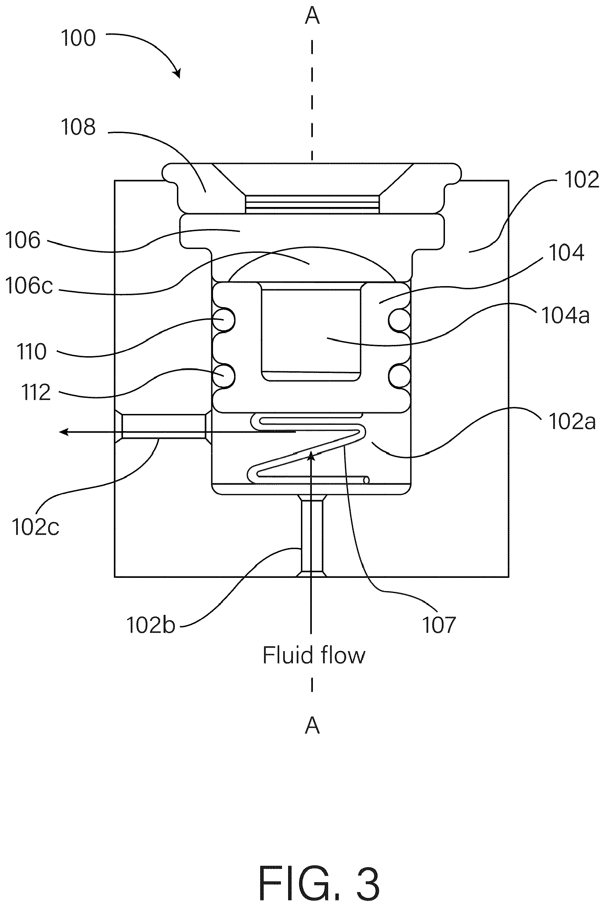

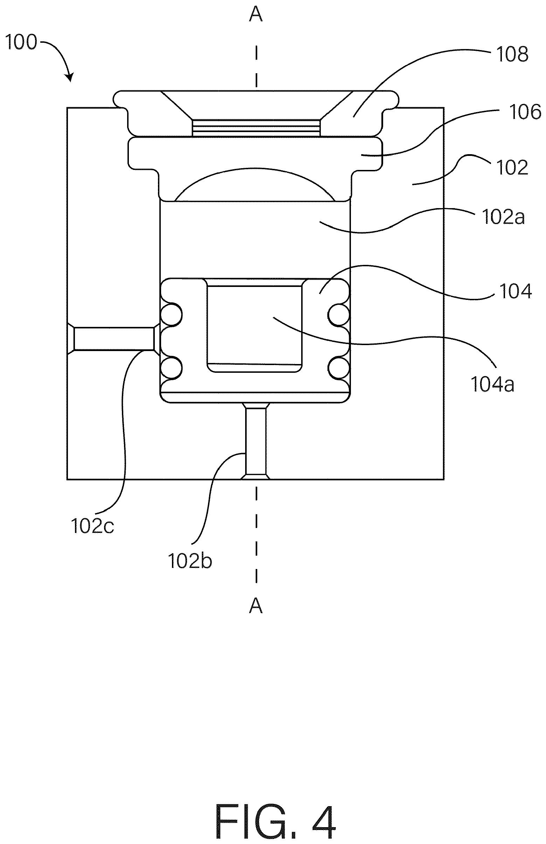

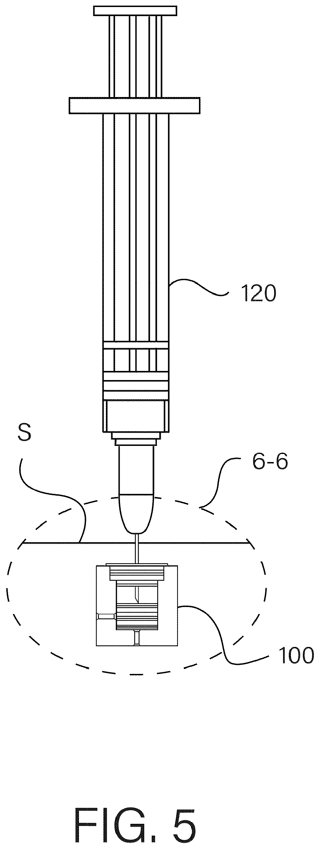

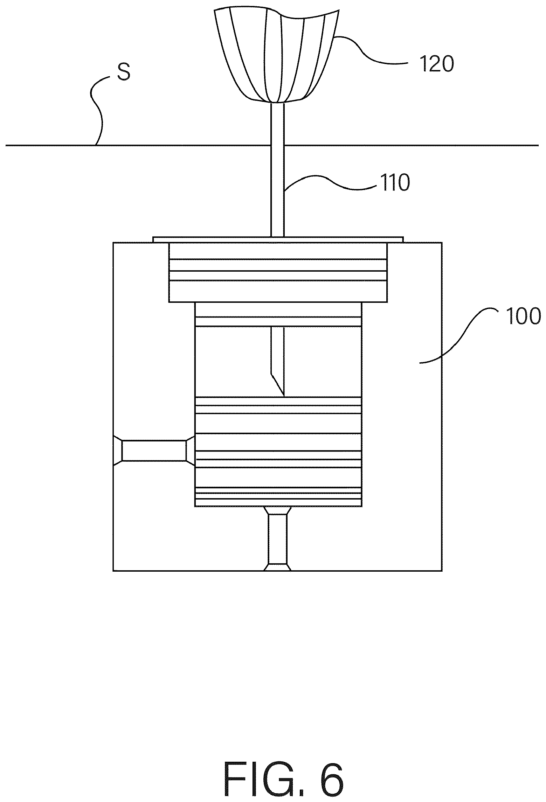

An example of a hydraulically actuated subcutaneous valve device 100 is shown and described with reference to FIGS. 1-6. A perspective view of the valve device 100 is shown in FIG. 1, and an exploded view of the valve device 100 is shown in FIG. 2. A cross-section view of the valve device 100 in an un-actuated (or open) state is shown in FIG. 3, and in an actuated (or closed) state is shown in FIG. 4. FIG. 5 show a cross-section view of the valve device 100 in an actuated (or closed) state, with a syringe needle extending into the valve device 100 for controlling the actuation or de-actuation of the valve device 100. FIG. 6 shows an enlarged view of the circled portion of FIG. 5.

The valve device 100 includes a valve body 102, a moveable member 104, a septum 106 and a needle guide 108. In other examples, the needle guide 108 may be omitted or may be formed integral with the valve body 102. The valve body 102 has a cavity 102a defining an inner volume in which the moveable member 104 and the septum 106 are located. The needle guide 108 is secured on a first end (the upper end in FIGS. 1-6) of the valve body 102. The first end of the valve body 102 (the upper end in FIGS. 1-6) opens to the cavity 102a, but is sealed by the septum 106.

As further described herein, the needle guide 108, the septum 106 and the first end of the valve body 102 define a port for receiving a portion of a needle of a syringe, smart pen or other external fluid injector. The fluid injector may be used to selectively inject or remove a valve operating fluid into or from a section of the inner volume of the cavity 102a (the upper section in FIGS. 3-6), through that port, to selectively actuate or de-actuate the valve device 100. The valve operating fluid (also referred to herein as the second fluid) may be any suitable fluid that may be selectively injected into and selectively removed from the hydraulically actuated valve device as described herein and, in particular examples, is a biologically inert fluid such as, but not limited to a saline solution.

The valve body 102 of the valve device 100 may be made of any suitably rigid and durable material such as, but not limited to metal, plastic, ceramic, composite material, combinations thereof or the like. In the illustrated example, the valve body 102 has a generally cylindrical shape with a round cross-section shape (on a cross-section perpendicular to the axis A of the generally cylindrical shape). In other examples, the valve body 102 may have other suitable shapes including, but not limited to, other cylindrical shapes with polygonal or non-round cross-section shapes, cubic or rectangular cubic shapes, spherical or partially spherical shapes, or the like. In further examples, the valve body 102 may be formed integral with other portions of a medical device and, thus, may have a shape at least partially determined by such other portions of the medical device.

The valve body 102 includes an inlet flow passage 102b and an outlet flow passage 102c, where each passage is in fluid flow communication with the inner volume of the cavity 102a. In FIG. 3, the inlet passage 102b extends vertically (such as, but not limited to axially) from an opening on one end (the bottom end in FIG. 3) of the valve body 102 to the inner volume of the cavity 102a. In addition, the outlet passage 102c extends transverse (such as, but not limited to perpendicular) to the axis A, from an opening on the sidewall of the valve body 102 to the inner volume of the cavity 102a.

The drawing in FIG. 3 includes arrows extending through the inlet and outlet passages 102b and 102c, to represent a direction of flow of the first fluid, when the valve device 102 is in an unactuated (or open) state. In other examples, the direction of flow of the first fluid may be opposite to that shown by the arrows in FIG. 3, such that the passage 102c is the inlet passage, and the passage 102b is the outlet passage. In yet other examples, the valve body 102 may have more than one inlet passage or more than one outlet passage (or more than one of each of those passages).

The movable member 104 is located within the cavity 102a of the valve body 102. The movable member 104 may include a unitary body or a multi-piece body, made of any suitably rigid and durable material such as, but not limited to metal, plastic, ceramic, composite material, combinations thereof or the like. The movable member 104 has an outer dimension and size that sufficiently matches (or is slightly smaller than) the inner dimension and size of the cavity 102a, to enable the movable member 104 to move as a piston by sliding in the axial direction A within the cavity 102a, relative to the valve body 102.

In certain examples, the moveable member 104 may have a generally cylindrical outer peripheral shape, and the cavity 102a of the valve body 102 may have a corresponding, cylindrical inner peripheral shape. In certain examples, the moveable member 104 has a cavity 104a and is open on one end (the upper end in FIGS. 2-6) to an inner volume space of the cavity 104a. In those examples, an end portion of a needle of a syringe, smart pen or other external fluid injector may be received in the inner volume space of the cavity 104a during a valve actuation or de-actuation operation, as described below. In other examples, the cavity 104a and the opening on the end of the moveable member 104 may be omitted.

The moveable member 104 may include one or more seals (two seals shown in FIGS. 2-6), to provide a fluid seal between the outer peripheral surface of the moveable member 104 and the inner surface of the cavity 102a. The two seals in the example in Figs. 2-6 are provided by two O-ring seal members 110 and 112 that reside in corresponding annular grooves 104b and 104c (shown in Fig. 2) on the movable member 104, and that may be made of a suitable silicon rubber, Teflon (trademark), neoprene, or other sealing material. In other examples, other suitable seals may be employed. In yet other examples, the seals may be omitted and a sufficient seal is provided by the engagement of the moveable member 104 with the inner surface of the cavity 102a.

The movable member 104 is movable within the cavity 102a between an unactuated (or open) position as shown in FIG. 3, and an actuated (or closed) position as shown in FIG. 4. In the unactuated (or open) position (FIG. 3), the moveable member 104 is located at one end (the upper end in FIG. 3) of the cavity 102a. In that position, a section of the inner volume of the cavity 102a that is in fluid flow communication with the inlet and outlet passages 102b and 102c, is located on one side (the lower side in FIG. 3) of the moveable member 104. Accordingly, when the movable member 104 is in the unactuated (or open) position (FIG. 3), the moveable member 104 does not obstruct flow through the valve body 102, from the inlet passage 102b, to the outlet passage 102c. In that state, the valve device 100 is open to enable fluid flow from the inlet channel 102b to the outlet channel 102c. Accordingly, in that state, a first fluid (such as, but not limited to a drug, treatment fluid, marker, biological fluid or other fluid) may flow through valve body 102, by flowing in through the inlet passage 102b, through the unobstructed section of the cavity 102a, and out through the outlet passage 102c, as represented by the arrows in FIG. 3.

In certain examples, the valve device 100 may include a bias mechanism 107 that applies a force to bias the moveable member 104 toward the septum 106 (to an unactuated (or open) position as shown in FIG. 3). The bias mechanism 107 may include, but is not limited to one or more springs arranged to bias the moveable member 104 toward the unactuated (or open) position in FIG. 3. For example, the bias mechanism may include a coil spring located adjacent one end of the moveable member 104 (the lower end in FIG. 3, which is the end facing the inlet channel), that biases the moveable member 104 toward the septum 106 (upward in FIG. 3). Alternatively or in addition, the moveable member 104 may be biased to move toward the septum 106 by fluid pressure from a fluid flow in through the inlet channel 102b (as represented by the arrow in the inlet channel 102b). Other examples may include other suitable bias mechanisms for biasing the moveable member 104 toward the septum 106 including, but not limited to magnetic attraction or repulsion by one or more permanent magnets or electromagnets located on the movable member 104 or on the valve body 102 (or in both).

In yet other examples, a bias mechanism (as discussed herein) may be arranged adjacent the other end of the moveable member 104 (the upper end in FIGS. 3 and 4), to bias the moveable member 104 in a direction away from the septum (toward the actuated or closed position as shown in FIG. 4. In those examples, the force applied by the bias mechanism may be overcome by a suitable fluid pressure from a fluid flow in through the inlet channel 102b (as represented by the arrow in the inlet channel 102b), to move the moveable member 104 toward the septum 106, to an un-actuated (or open) position.

The septum 106 is configured to be pierced, transcutaneously, by a needle of a syringe, smart pen or other external fluid injector (as shown in FIGS. 5 and 6), while the valve device 100 (or a medical device containing the valve device 100) is located in a subcutaneous implant site, in a patient. In that manner, the fluid injector may transcutaneously inject or remove an operating fluid from the valve device 100, as described herein, to selectively change the state of the valve device 100 between the un-actuated (or open) valve state and the actuated (or closed) valve state.

The septum 106 provides a fluid seal, to seal one end of the cavity 102a, and may be made of a material that is configured to be pierced by the needle of a syringe, smart pen or other fluid injector. The septum 106 is also configured to provide a fluid seal around the needle of the fluid injector, and to reseal after removal of the needle. The septum 106 may be made of any suitable material for such purposes, such as, but not limited to silicone rubber, polyurethane, or other elastic polymer, or the like. In some examples, the septum 106 may be pre-pierced or formed with a slot or cut that is configured to self-seal around a needle, and to self-seal after removal of the needle.

The septum 106 has a configuration and shape to fit partially or fully into the cavity 102a of the valve body 102. In the example in FIGS. 2-6, the septum 106 has a first section 106a (the lower section in FIGS. 2-6) with a first diameter, and a second section 106b (the upper section in FIGS. 2-6) with a second diameter, where the first diameter 106a is smaller than the second diameter 106b. In that example, the cavity 102a has a section (the lower section in FIGS. 3-6) with a corresponding first diameter about equal to or slightly smaller or slightly larger than the first diameter 106a of the septum 106. The first diameter of the cavity 102a may correspond to the diameter of the section of the cavity 102a in which the moveable member 104 moves. The cavity 102a in that example has a further section (the upper section in FIGS. 3-6) with a corresponding second diameter about equal to or slightly smaller or slightly larger than the second diameter 106b of the septum 106.

Accordingly, the larger, second diameter section 106b of the septum 106 abuts the valve body 102 and retains the septum in a fixed location in the cavity 102a. In the example in FIGS. 2-6, the septum 106 and the cavity 102a have a stepped configuration, where the diameter changes abruptly from the first diameter to the second diameter. In other examples, the septum 106 or the cavity 102a (or both) may have a tapered or partially conical shape that changes continuously from the first diameter to the second diameter.

The septum 106 has a surface (the bottom surface in FIGS. 2-6) that faces toward the moveable member 104. In the example in FIGS. 2-6, that surface of the septum 106 has a concave or dome shape and defines a volume 106c within the concavity. The volume 106c may be configured with a sufficient depth along the axial dimension A to receive an end portion and the fluid opening of the needle of the fluid injector device. In some examples, such as those in which the moveable member 104 does not have an open end to an inner cavity 104c, the volume 106c defined by the septum can provide sufficient clearance to enable an operating fluid to be injected into the cavity 102a from the needle of an injector device, while the movable member 104 is in the unactuated (or open) position of FIG. 3

The needle guide 108 may be provided to help guide the needle of the fluid injector device toward a central portion of the septum 106. In certain examples, the needle guide 108 includes an annular body having a central opening 108a. The annular body may have a tapered or partially cone shaped surface 108b that tapers from a large diameter open end of the annular body (the upper end in FIGS. 2-6), toward the smaller diameter central opening 108a.

In certain examples, the needle guide 108 has a configuration and shape to fit partially or fully into the cavity 102a of the valve body 102. In the example in FIGS. 1-6, a first section 108c of the needle guide 108 is located outside of the cavity 102a and has a diameter larger than the cavity 102a and forms a lip or flange that inhibits the needle guide 108 from being pushed into the cavity 102a. Also in that example, the needle guide 108 has a second section 108d that extends at least partially into the cavity 102a of the valve body and abuts the septum 106.

The needle guide 108 may be made of any suitably rigid and durable material that resists puncture from a needle, such as, but not limited to metal, plastic, ceramic, composite material, combinations thereof or the like. The needle guide 108 may be secured to the valve body 102 by any suitable securing mechanism such as, but not limited to press or friction fitting, adhesive material, welding, heat staking, combinations thereof, or the like. In particular examples, the needle guide 108 retains or helps to retain the septum 106 within the cavity 102a of the valve body 102.

In certain examples, the valve device 100 may be made by providing a valve body 102 having an open cavity 102b and fluid inlet and outlet passages in fluid flow communication with the cavity 102b, and assembling the other components with the valve body 102. For example, the moveable member 104 may be inserted into the cavity 102b and, then, the septum may be inserted into the cavity 102b over the moveable member 104, to close one end of the cavity 102b. Then the needle guide 108 may be secured to the valve body 102, over the septum 106. In particular examples, the needle guide 108 may be pressed against the septum 106, with sufficient force to urge the septum 106 to expand outward in a lateral direction against the valve body 102 and provide or enhance the fluid seal between the septum 106 and the valve body 102.

As described herein, the valve device 100 may be employed in a subcutaneous implant environment in which the valve device 100 (or a medical device or system having the valve device 100) is implanted at a subcutaneous implant site in a patient. Any suitable implant method may be employed to implant the valve device 100 (or medical device or system). Once the valve device 100 is implanted, the valve device 100 may be operated, transcutaneously, to selectively actuate (close) or de-actuate (open) the valve device 100.

In particular, with reference to FIGS. 5 and 6, a needle 110 of a syringe, smart pen or other transcutaneous fluid delivery device 120 may be inserted through the skin S of the patient and through the septum 106 (or into the volume 106c formed by the septum 106) of the implanted valve device 100. To actuate the valve device 100 to an actuated (or closed) state, an operating fluid may be injected from the fluid delivery device 120, into the cavity 102a of the valve body 102, adjacent one end (the upper end in FIGS. 2-6) of the moveable member 104. An amount of the operating fluid may be injected into the cavity 102a to provide a fluid pressure in a section of the cavity 102a adjacent that end of the moveable member 104 sufficient to force the moveable member 104 to move from the un-actuated position (or open position) of FIG. 3 to an actuated (or closed) position of FIG. 4-6. As that section of the cavity 102a is sealed from the inlet and outlet channels by the moveable member 104, the fluid pressure remains within that section of the cavity 102a, to maintain the valve device 100 in an actuated (or closed) state, after removal of the needle 110. In some examples, the needle 110 may contact and impart a pressing force on the moveable member 104, to move the moveable member 104 toward the actuated (closed) position, and the fluid pressure of the operating fluid may retain the moveable member 104 in the actuated (closed) position after removal of the needle.

To de-actuate the valve device 100 from an actuated state to a unactuated state, the syringe, smart pen or other transcutaneous fluid delivery device 120 may be transcutaneously inserted into the valve device 100 (as shown in FIGS. 5 and 6) and then operated to withdraw a sufficient amount of the operating fluid from the cavity 102a, to reduce a fluid pressure in the section of the cavity 102a adjacent the end of the moveable member 104, and enable the moveable member 104 to move from the actuated position (or closed position) of FIGS. 4-6 to an unactuated (or open) position of FIG. 3. As discussed above, in certain examples, the valve device 100 may include a spring or other bias device 107 to move the moveable member 104 toward the unactuated (or open) position, when the fluid pressure of the operating fluid is sufficiently reduced. In other examples, the pressure of fluid flow through inlet channel and the reduction of pressure from the operating fluid causes the moveable member 104 to move toward the unactuated (or open) position, when the fluid pressure of the operating fluid is sufficiently reduced.

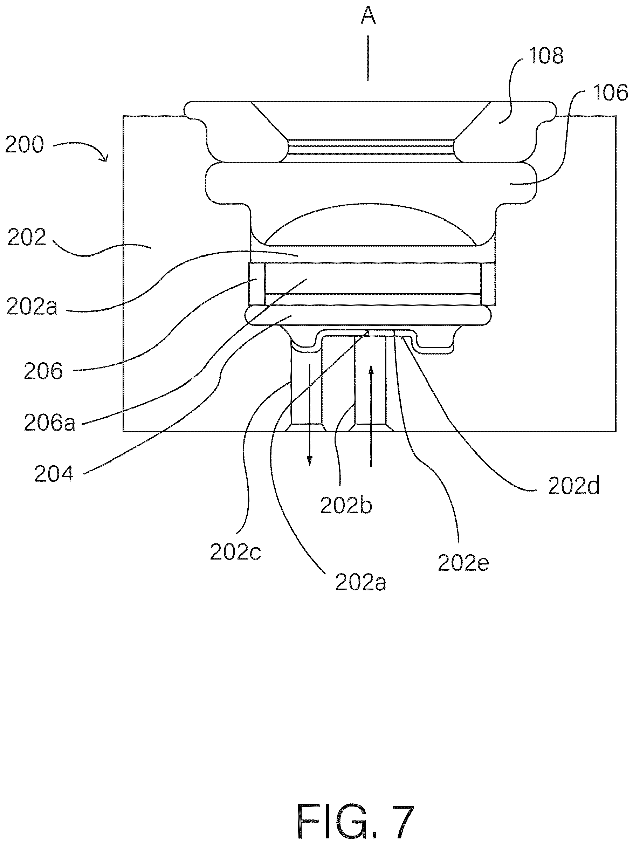

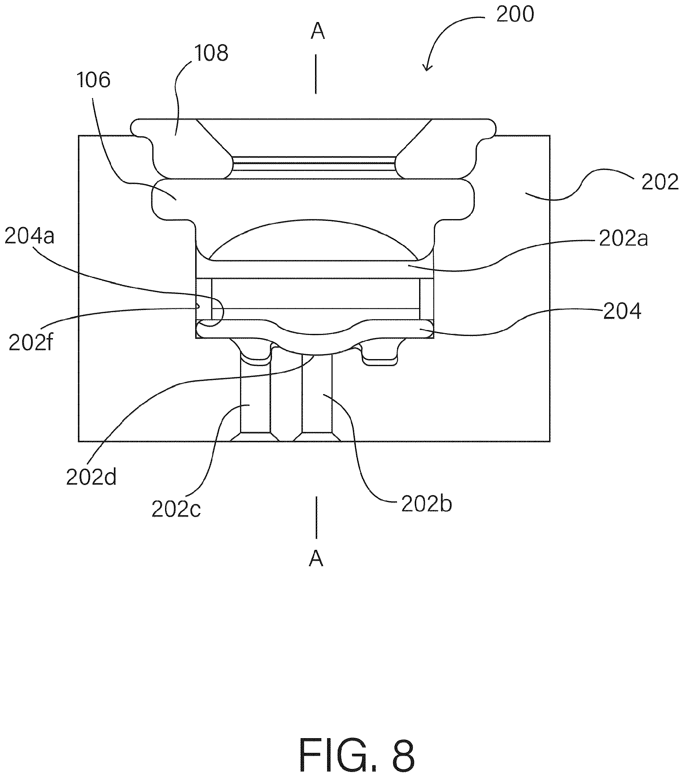

Another example of a hydraulically actuated subcutaneous valve device 200 is shown and described with reference to FIGS. 7-8. More specifically, FIG. 7 shows a cross-section view of the valve device 200 in an un-actuated (or open) state, and FIG. 8 shows a cross-section view of the valve device 200 in an actuated (or closed) state. The valve device 200 (and components thereof) corresponds to the valve device 100 (and corresponding components thereof) described herein, except for the differences described below. Components of the valve device 200 that correspond in structure and function to components of the valve device 100 are provided with corresponding reference numbers.

In particular, the valve device 200 includes a septum 106 and a needle guide 108, corresponding to the septum and needle guide of the valve device 100. The valve device 200 also includes a valve body 202 having a cavity 202a that generally correspond to the valve body 102 and cavity 102a of the valve device 100. In other examples, the needle guide 108 may be omitted or may be formed integral with the valve body 202.

The valve body 202 has a fluid inlet channel 202b and a fluid outlet channel 202c that correspond, generally, to the fluid inlet channel 102b and the fluid outlet channel 102c of the valve body 102. However, the fluid inlet channel 202b and the fluid outlet channel 202c of the valve body 202 each extend through one end (the lower end in FIGS. 7 and 8) of the valve body 202. In certain examples, one or each of the fluid inlet and fluid outlet channels 202a and 202b may be parallel to the axis A of the valve body 202.

The valve device 200 need not include a moveable member (e.g., moveable member 104 of the valve device 100). Instead, the valve device 200 includes a hydraulically deformable diaphragm 204. In addition, the valve body 202 of the valve device 200 includes a valve seat surface 202d located around one or both of the fluid inlet and fluid outlet channels 202b and 202c.

In some examples, the deformable diaphragm 204 is supported within the cavity 202a of the valve body 202 at a position to provide a gap 202e between the deformable diaphragm 204 and the valve seat surface 202d, when the valve device 200 is in an un-actuated (or open) state, as shown in FIG. 7. The deformable diaphragm 204 is configured to deform and engage the valve seat surface 202d to close one or both of the fluid inlet channel 202b and the fluid outlet channel 202c of the valve body 202, when the valve device 200 is in an actuated (or closed) state, as shown in FIG. 8. The deformable diaphragm 204 is configured to elastically return to its un-deformed shape, when the valve device 200 is in a de-actuated (or open) state.

In other examples, the gap 202e may be omitted and the deformable diaphragm 204 may be arranged to be in contact with, or press against the valve seat surface 202d, when the valve device 200 is in an un-actuated (or open) state and no (or insufficient) fluid pressure is provided from the fluid inlet channel 202b. In those examples, when a sufficient fluid pressure (or cracking pressure) is provided by the first fluid in the fluid inlet channel 202b, that fluid pressure may move or deform the deformable diaphragm 204, enabling the first fluid to flow into the cavity 202a of the valve body and out of the fluid outlet channel 202c.

The valve device 200 may be selectively actuated in the same manner as described above with regard to actuating the valve device 100, by selectively injecting an operating fluid into the cavity 202a from a syringe, smart pen or other transcutaneous fluid delivery device 120. Similarly, the valve device 200 may be selectively de-actuated from an actuated state in the same manner as described above with regard to de-actuating the valve device 100, by withdrawing operating fluid from the cavity 202a. Accordingly, the valve device 200 may be selectively actuated and de-actuated, transcutaneously, as described above with regard to the valve device 100.

The deformable diaphragm 204 may be made of any suitable deformable and durable material such as, but not limited to a deformable, elastic plastic, rubber, ceramic, metal, composite material, combinations thereof, or the like. The outer peripheral edge portion 204a of the deformable diaphragm 204 may be secured to the inner peripheral surface 202f of the cavity 202a of the valve body 202 by any suitable securing mechanism including, but not limited to press or friction fitting, adhesive material, welding, heat staking, combinations thereof, or the like. In certain examples, one or both of the deformable diaphragm 204 and the valve seat surface 202d may be configured to interact with each other as a needle valve, a ball valve or other suitable valve configuration and may function as a check valve, a burst valve or other type of valve.

In certain examples, the valve device 200 also includes a needle guard member 206 located in the cavity 202a, between the septum 106 and the deformable diaphragm 204. The needle guard member 206 is configured to protect the deformable diaphragm 204 from puncture by the needle of the syringe, smart pen or other transcutaneous fluid delivery device 120, during an actuation or a de-actuation operation. The needle guard member 206 is further configured to enable operating fluid to flow through and apply hydraulic pressure on the deformable diaphragm. In some examples, the needle guard member 206 may have an open cavity 206a on the end/side facing the septum 106, for receiving an end portion of a needle, similar to the function of the cavity 104a of the moveable member 104.

The needle guard member 206 may be made of any sufficiently rigid and durable material that may be engaged by the needle of the syringe, smart pen or other transcutaneous fluid delivery device 120, and resist a puncture through the needle guard member 206 by the needle. The needle guard member 206 may include one or more (or a plurality of) fluid flow passages, that enable operating fluid to flow through or past the needle guard member 206. In particular examples, the needle guard member 206 is made of a metal, plastic, ceramic, composite material, combinations thereof, or the like.

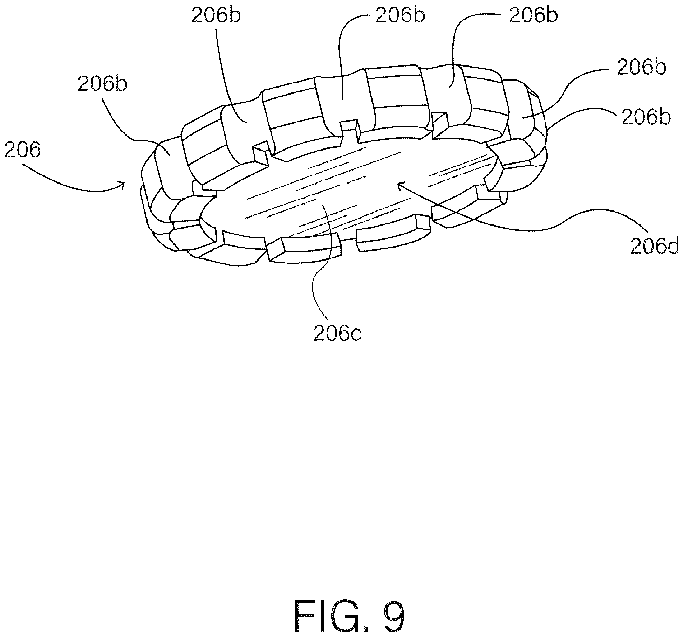

An example of a needle guard member 206 is shown in FIG. 9. In that example, the needle guard member 206 has a generally disc-shaped configuration, with a plurality of channels 206b that act as fluid flow passages provided around the outer periphery of the needle guard member 206. The channels 206b enable the valve operating fluid to pass the needle guard member 206 and impart fluid pressure on the hydraulically deformable diaphragm 204, when operating fluid is injected into the cavity 202a. The central portion 206c of the needle guard member 206 is free of channels, to engage and inhibit passage of the needle of the syringe, smart pen or other transcutaneous fluid delivery device 120. In other examples, one or more (or each) of the fluid flow channels may be provided in other suitable locations on the needle guard member 206.

In certain examples, as shown in FIG. 9, the peripheral edge of the needle guard member 206 extends axially A beyond the central portion 206c of the needle guard member 206, to form a recess 206d on the side/end of the needle guard member 206 that faces the deformable diaphragm 204 (the bottom side/end in FIGS. 7-9). In those examples, the peripheral edge portion of the needle guard member 206 may engage and retain (or help retain) the diaphragm 204 in place within the cavity 202a of the valve body, while the recess 206c provides a gap between the central portion of the needle guard member 206 and the diaphragm 204. The gap between the central portion of the needle guard member 206 and the diaphragm 204 provides a volume space in which valve operating fluid injected into the cavity 202a may collect and act (provide fluid pressure) on the diaphragm 204. The needle guard member 206 may be secured to the valve body 202 in any suitable manner including, but not limited to press or friction fitting, adhesive material, welding, heat staking, combinations thereof, or the like.

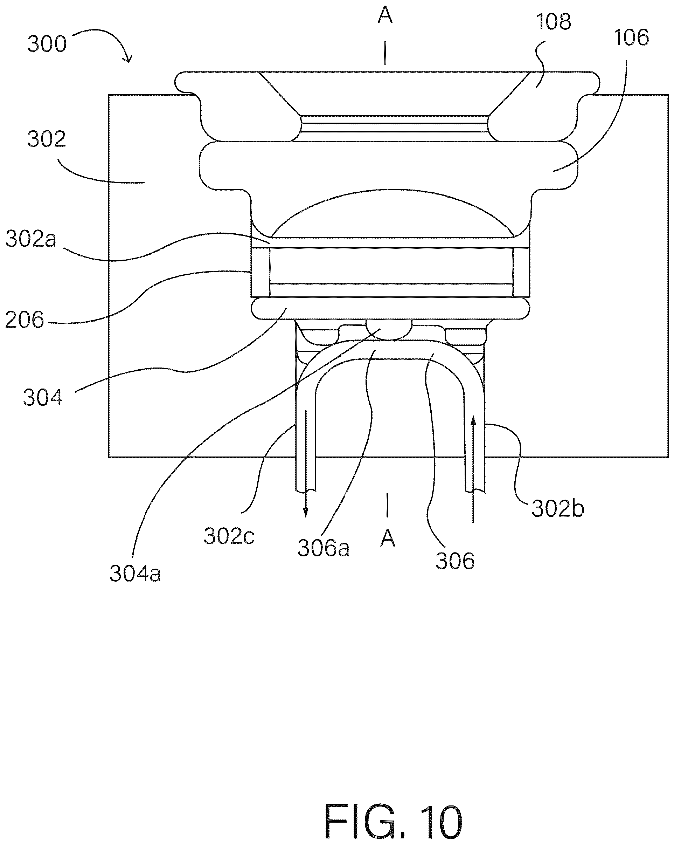

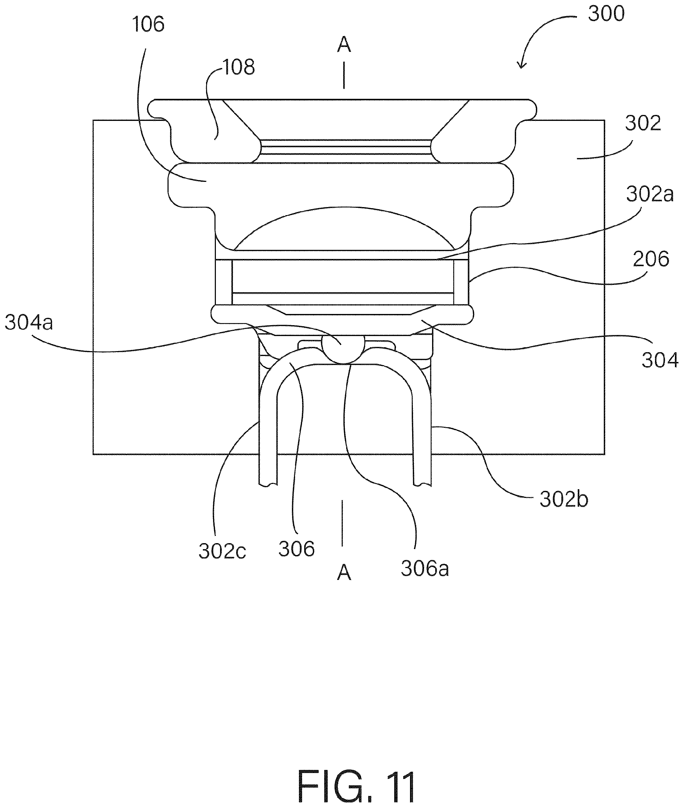

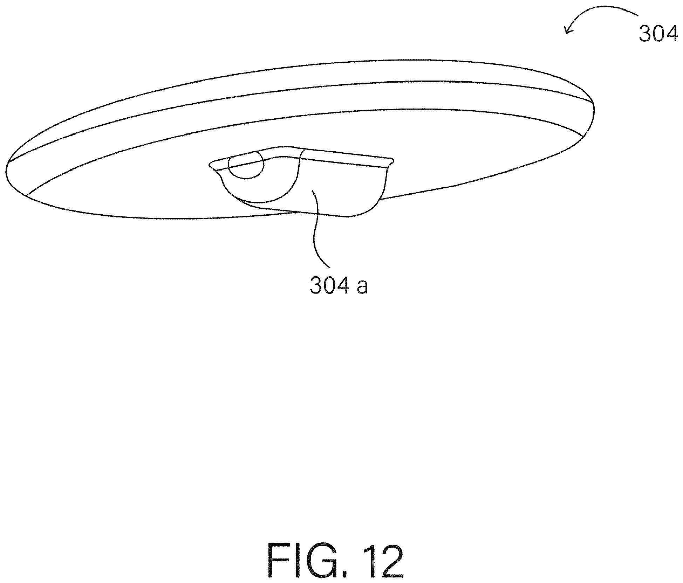

Another example of a hydraulically actuated subcutaneous valve device 300 is shown and described with reference to FIGS. 10-12. More specifically, FIG. 10 shows a cross-section view of the valve device 300 in an un-actuated (or open) state, and FIG. 11 shows a cross-section view of the valve device 300 in an actuated (or closed) state. An example of a deformable diaphragm for the valve device 300 is shown in FIG. 12. The valve device 300 (and components thereof) corresponds to the valve device 200 (and corresponding components thereof) described herein, except for the differences described below. Components of the valve device 300 that correspond in structure and function to components of the valve device 200 or 100 are provided with corresponding reference numbers.

In particular, the valve device 300 includes a septum 106 and a needle guide 108, corresponding to the septum and needle guide of the valve device 200. The valve device 300 also includes a valve body 302 having a cavity 302a that generally correspond to the valve body 202 and the cavity 202a of the valve device 200. In other examples, the needle guide 108 may be omitted or may be formed integral with the valve body 302. In particular examples, the valve device 300 may include a needle guard member 206 corresponding to the needle guard member of the valve device 200.

The valve device 300 also includes a hydraulically deformable diaphragm 304 that is similar to the hydraulically deformable diaphragm 204 of the valve device 200. However, the hydraulically deformable diaphragm 304 includes an obstruction feature 304a that shifts between an unactuated (or open) position shown in FIG. 10 where the diaphragm 304 is not deformed, and an actuated (or closed) position shown in FIG. 11 where the diaphragm 304 is deformed by operating fluid pressure. In particular examples, the obstruction feature 304a is formed integral with the rest of the hydraulically deformable diaphragm 304 for example, by being molded or co-molded with the hydraulically deformable diaphragm 304. In other examples, the obstruction feature 304a may be formed separate from and secured to the rest of the hydraulically deformable diaphragm 304 by any suitable securing mechanism such as, but not limited to adhesive material, welding, heat staking, combinations thereof, or the like.

The valve device 300 has a fluid inlet channel 302b and a fluid outlet channel 302c that correspond, generally, to the fluid inlet channel 202b and the fluid outlet channel 202c of the valve body 202. However, in the valve device 300, a flexible fluid flow tubing 306 extends through the fluid inlet channel 302b, across a portion of an inner surface (the bottom surface in FIGS. 10 and 11) of the cavity 302a, and through the fluid outlet channel 302c. The flexible fluid flow tubing 306 may be made of any suitable, flexible material such as, but not limited to a polyether block amide (PEBA) of thermoplastic elastomer (TPE) such as PEBAXTM, a polytetrafluoroethylene (PTFE), an ethylene tetrafluoroethylene (ETFE), a thermoplastic polyurethane (TPU) such as PELLETHAN^{™}, a fluorinated ethylene propylene (FEP) or other fluoropolymer, an ethylene vinyl acetate (EVA), a silicone material or the like. However, for other contexts and applications of use, the tubing 306 may be made of other materials suitable and compatible with those contexts and applications. In certain examples, the tubing 306 may be consistent and uniform in flexibility along its length. In other examples, the tubing 306 may have a central section 306a that has a greater flexibility relative to other sections of the tubing 306 within the fluid inlet and outlet channels 302b and 302c. The central section 306a of the tubing 306 may have an enhanced flexibility relative to other sections of the tubing by, for example, providing or forming the central section 306a with a different material having greater flexibility than the material of the other sections of the tubing 306, by providing or forming a wall of the tubing in the central section 306a to be thinner than the other sections of the tubing 306, by providing or forming a wall of the tubing in the central section 306a to have a shape or other configuration that enhances flexibility relative to other sections of the tubing 306, or by any combination thereof.

When the valve device 300 is in the unactuated (or open) state as shown in FIG. 10, the flexible tubing 306 is sufficiently unobstructed to enable first fluid to flow through the tubing. However, when the valve device is in the actuated (or closed) state as shown in FIG. 11, the hydraulically deformable diaphragm 304 deforms a sufficient amount to press the obstruction feature 304a against the flexible tubing 306 and obstruct or shut off first fluid flow through the flexible tubing 306. In particular examples, the obstruction feature 304a is configured to compress and pinch the flexible tubing 306 between the obstruction feature 304a and the inner surface of the cavity 302a, when in the actuated (or closed) position shown in FIG. 11.

The valve device 300 may be selectively actuated in the same manner as described above with regard to actuating the valve devices 100 and 200, by selectively injecting an operating fluid into the cavity 302a from a syringe, smart pen or other transcutaneous fluid delivery device 120. Similarly, the valve device 300 may be selectively de-actuated from an actuated state in the same manner as described above with regard to de-actuating the valve devices 100 and 200, by withdrawing operating fluid from the cavity 302a. Accordingly, the valve device 300 may be selectively actuated and de-actuated, transcutaneously, as described above with regard to the valve devices 100 and 200.

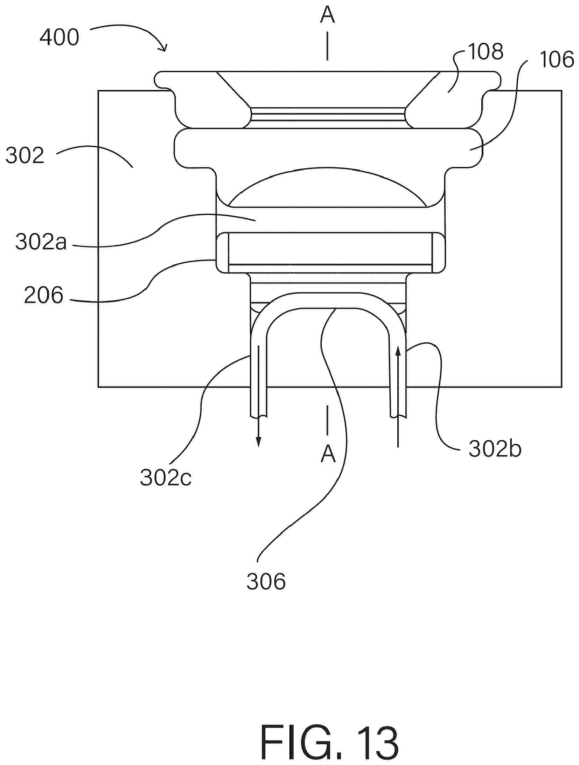

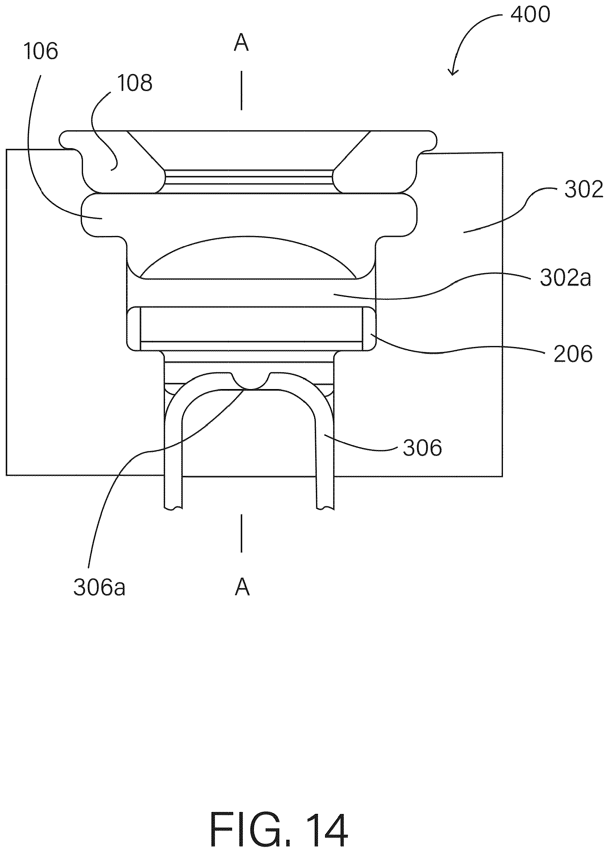

Another example of a hydraulically actuated subcutaneous valve device 400 is shown and described with reference to FIGS. 13-14. More specifically, FIG. 13 shows a cross-section view of the valve device 400 in an un-actuated (or open) state, and FIG. 14 shows a cross-section view of the valve device 400 in an actuated (or closed) state. The valve device 400 (and components thereof) corresponds to the valve device 300 (and corresponding components thereof) described herein, except that the valve device 400 does not include the hydraulically deformable diaphragm 304. Components of the valve device 400 that correspond in structure and function to components of the valve device 300, 200 or 100 are provided with corresponding reference numbers.

In particular, the valve device 400 includes a septum 106 and a needle guide 108, corresponding to the septum and needle guide of the valve device 100, 200 or 300. The valve device 400 also includes a valve body 302 having a cavity 302a that generally correspond to the valve body 302 and the cavity 302a of the valve device 300. In other examples, the needle guide 108 may be omitted or may be formed integral with the valve body 302. The valve device 400 also includes a flexible tubing 306 corresponding to the flexible tubing 306 of the valve device 300. In particular examples, the valve device 400 may include a needle guard member 206 corresponding to the needle guard member of the valve device 200 or 300, for protecting the flexible tubing 306 from being punctured by the needle of the syringe, smart pen or other transcutaneous fluid delivery device 120.

As noted above, the valve device 400 does not include a hydraulically deformable diaphragm between the needle guard member 206 and the flexible tubing 306. Instead, the flexible tubing 306 is configured to be sufficiently flexible to flex between an unactuated (or open) state as shown in FIG. 13, and an actuated (or closed) state as shown in FIG. 14 in response to the pressure from the operating fluid itself. In the unactuated (or open) state, the flow channel of the flexible tubing 306 is sufficiently unobstructed to enable first fluid flow through the flexible tubing 306. In the actuated (or closed) state, the flexible tubing 306 is sufficiently compressed or pinched to obstruct or cut-off first fluid flow through the flexible tubing 306.

In the valve device 400, the flexible tubing is configured to be sufficiently compressed or pinched to be in an actuated (or closed) state, by fluid pressure of a sufficient amount of operating fluid injected into the cavity 302a of the valve body 302. In the valve device 400, the flexible tubing 306 is configured to be in (or elastically return to) an uncompressed state, when the operating fluid is not present (or is withdrawn from) the cavity 302a. In certain examples, the flexible tubing 306 of the valve device 400 (or of the valve device 300) may have a generally round cross-section shape, when in the uncompressed state. In yet other examples, the flexible tubing 306 may have an oval or generally flattened cross-section when in the uncompressed state, to be able to collapse and compress at a lower pressure than a round cross-section tubing. In yet other examples, the walls of the flexible tubing 306 may be thinner at the section to be compressed to enable a lower compression force from the operating fluid to close off that section of flexible tubing 306.

The valve device 400 may be selectively actuated in the same manner as described above with regard to actuating the valve device 300, by selectively injecting an operating fluid into the cavity 302a from a syringe, smart pen or other transcutaneous fluid delivery device 120. Similarly, the valve device 400 may be selectively de-actuated from an actuated state in the same manner as described above with regard to de-actuating the valve devices 100 and 200, by withdrawing operating fluid from the cavity 302a. Therefore, the valve device 400 may be selectively actuated and de-actuated, transcutaneously, as described above with regard to the valve devices 100, 200 and 300.

As described herein, one or more valve devices 100, 200, 300 or 400 may be included in an implantable medical device or system. In some examples, the implantable medical device may include an implantable infusion pump that is configured to infuse controlled amounts of an infusion media, such as a drug, treatment fluid, marker, or other fluid, to the patient. In particular examples, the infusion media is insulin or an insulin formulation. In other examples, the infusion media is a cancer treatment drug, an AIDS treatment drug, a pain reducing medication, or drug for other types of treatment of the patient.

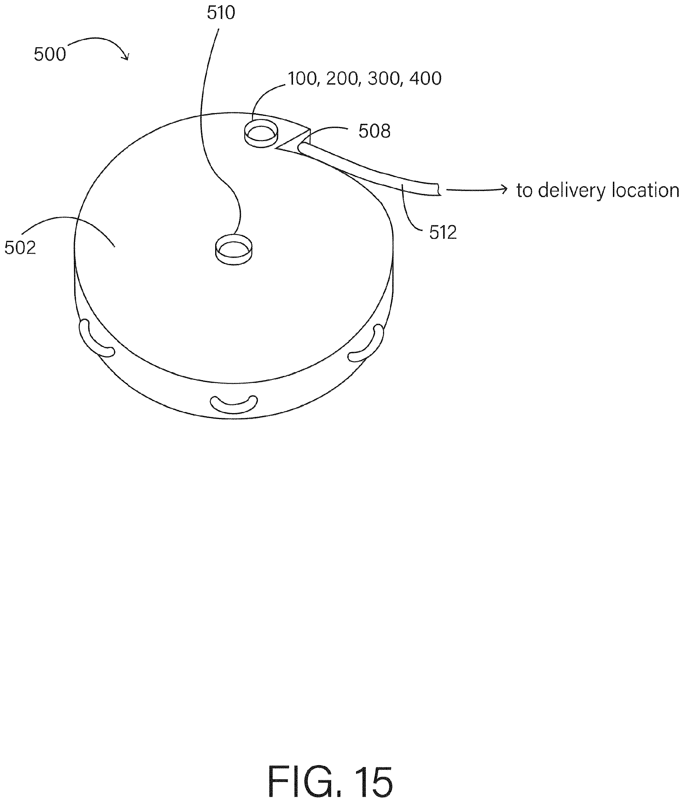

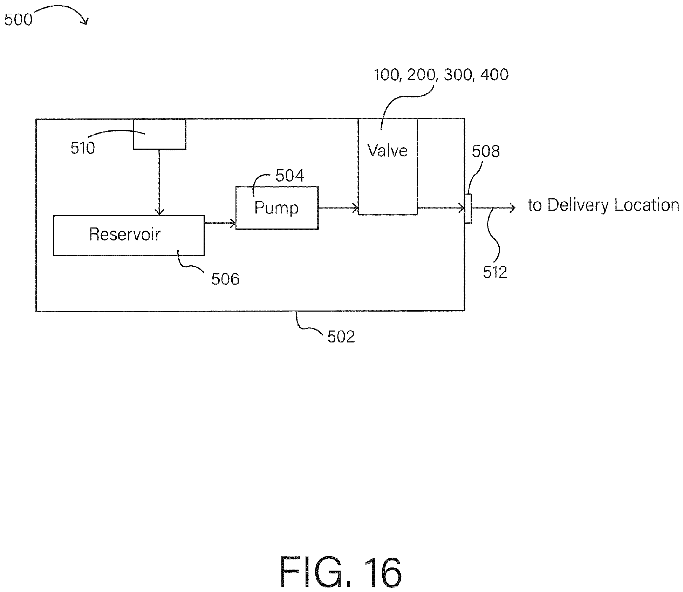

An example of an infusion pump 500 having a valve device 100, 200, 300 or 400 is shown in FIG. 15 and a schematic diagram of the infusion pump 500 is shown in FIG. 16. The infusion pump 500 includes a pump housing 502 containing an electronic pump device 504, a reservoir 506 for containing infusion media and an outlet port 508. The infusion pump 500 may include other components, such as, but not limited to a refilling port 510 for the reservoir. The pump device 504 may be configured to selectively draw infusion media from the reservoir 506 and deliver the infusion media to the outlet port 508 under sufficient pressure to drive the infusion media through a catheter 512 connected to the outlet port 508, for delivery to a desired location in the patient.

The arrow-lines in FIG. 16 represent a flow path for infusion media in the infusion pump 500. In the example in FIG. 16, when the valve device 100, 200, 300 or 400 is in an unactuated (or open) state, first fluid from the pump device 504 may be conveyed through the flow path to the inlet channel 102b, 202b or 302b of the valve device 100, 200, 300 or 400. In addition, infusion media from the outlet channel 102c, 202c or 302c of the valve device 100, 200, 300 or 400 may be conveyed through the flow path to the outlet port 508 of the infusion pump 500.

As shown in FIGS. 15 and 16, the infusion pump 500 also includes a hydraulically actuated valve device 100, 200, 300 or 400 located in the flow path for the infusion media, for example, between the pump device 504 and the outlet port 508. In that arrangement, the valve device 100, 200, 300 or 400 may be selectively actuated to close the infusion media flow path to the outlet port 508. In other examples, a valve device 100, 200, 300 or 400 be located in one or more other portions of the fluid flow path of the infusion pump 500.

In certain examples, the infusion media flow path of the infusion pump 500 is selectively closed by selectively actuating the valve device 100, 200, 300 or 400, as part of a flushing operation. In such examples, the valve device 100, 200, 300 or 400 is actuated to be in a closed state as described herein, to close the fluid flow path at or before the outlet port 508. With the fluid flow path in the infusion pump 500 closed and isolated from the patient, a flushing media may be injected into the infusion pump 500, through the refilling port 510. The flushing media may be communicated through the fluid flow paths, the reservoir and the pump (or through other components) of the infusion pump 500, to provide cleaning or flushing function. The flushing media may be any suitable fluidic media that provides a cleaning function, a flushing function or other suitable function associated with the infusion pump 500. In certain examples, the flushing media is a sodium hydroxide solution, an aqueous and organic solution containing salts, buffers, surface active agents, or solvents, or other suitable cleaning of flushing fluid.

During that flushing period, the valve device 100, 200, 300 or 400 may remain actuated (or closed), to inhibit passage of flushing fluid out of the outlet port 508. Once a suitable period of time of flushing of the infusion pump 500, the flushing media may be removed from the infusion pump 500, for example, by withdrawing the flushing media through the refilling port 510. Thereafter, the reservoir 506 may be sufficiently filled or re-filled with an infusion media. Once the flushing fluid has been removed, the valve device 100, 200, 300 or 400 may be de-actuated (or opened) to open the fluid flow path between the pump 504 and the outlet port 508.

After the reservoir 506 is sufficiently filled or re-filled with an infusion media, the infusion pump 500 may be selectively operated to selectively draw infusion media from the reservoir and deliver the infusion media to the outlet port, as described above. Accordingly, the valve device 100, 200, 300 or 400 may be selectively actuated for a flushing operation and, then, selectively de-actuated after completion of the flushing operation. In other examples, the valve device 100, 200, 300 or 400 may be selectively actuated (closed) and selectively de-actuated (opened) for purposes other than flushing.

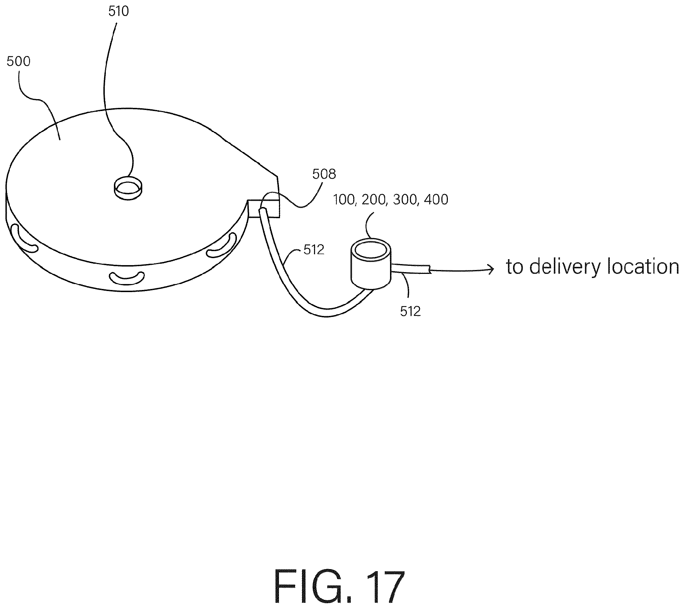

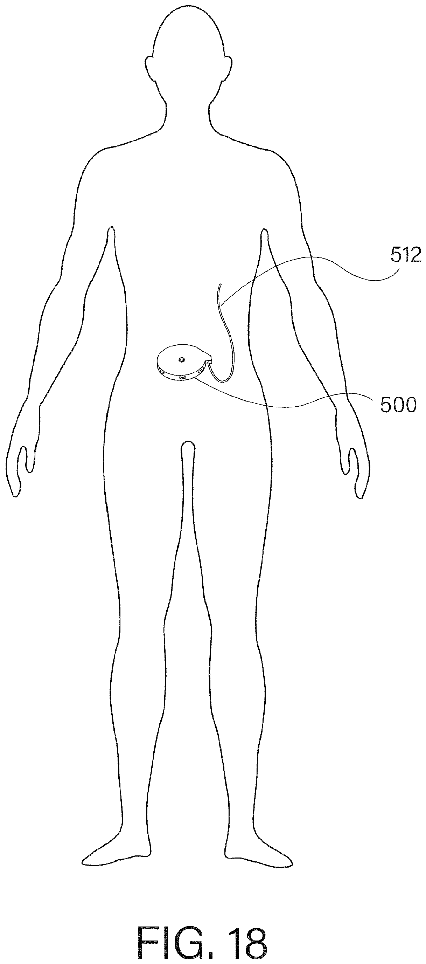

In the example in FIGS. 15 and 16, the valve device 100, 200, 300 or 400 is included in the infusion pump 500. In other examples, the valve device 100, 200, 300 or 400 may be separate from the infusion pump 500, and may be connected in or to the outlet catheter 512, as shown in the system of FIG. 17. In those or other examples, the infusion pump 500 and the valve device 100, 200, 300 or 400 may be implanted in a patient, as shown in FIG. 18.

While various exemplary embodiments have been presented in the foregoing detailed description, it should be appreciated that a vast number of variations exist. It should also be appreciated that the exemplary embodiment or embodiments described herein are not intended to limit the scope, applicability, or configuration of the claimed subject matter in any way. Rather, the foregoing detailed description will provide those skilled in the art with a convenient road map for implementing the described embodiment or embodiments. It should be understood that various changes can be made in the function and arrangement of elements without departing from the scope defined by the claims, which includes known equivalents and foreseeable equivalents at the time of filing this patent application.

Further disclosed herein is the subject-matter of the following clauses:
1. A hydraulically actuated valve device, comprising:
   a valve body having an inner cavity, an inlet channel extending to the inner cavity, an outlet channel extending from the inner cavity, and an open end that opens to the inner cavity;
   a septum located in the inner cavity, the septum being arranged to seal the open end of the valve body and being configured to receive a needle through which an operating fluid may be selectively injected into the inner cavity of the valve body to increase a fluid pressure in the inner cavity of the valve body, or selectively withdrawn from the inner cavity to reduce the fluid pressure in the inner cavity;
   a valve closing member arranged in the inner cavity of the valve body, the valve closing member having an unactuated state to enable a first fluid to flow from the inlet channel to the outlet channel when the fluid pressure of operating fluid in the inner cavity of the valve body is below a closing pressure, the valve closing member having an actuated state that inhibits the first fluid from flowing into the inner cavity from the inlet channel or out of the inner cavity through the outlet channel when the operating fluid in the inner cavity of the valve body is at or above the closing pressure.
2. The hydraulically actuated valve device of clause 1, wherein the valve closing member comprises a moveable member that is configured to be in a first position within the inner cavity of the valve body when the fluid pressure of operating fluid in the inner cavity of the valve body is below the closing pressure, and to move to a second position within the inner cavity of the valve body when the fluid pressure of the operating fluid in the inner cavity of the valve body is at or above the closing pressure, wherein the moveable member enables the first fluid to flow from the inlet channel to the outlet channel when the moveable member is in the first position, and wherein the moveable member obstructs at least one of the inlet channel and the outlet channel to inhibit flow of the first fluid from the inlet channel to the outlet channel when the moveable member is in the second position.
3. The hydraulically actuated valve device of clause 2, wherein the moveable member includes at least one seal member that provides a fluid seal between the moveable member and an inner wall of the cavity of the valve body.
4. The hydraulically actuated valve device of clause 3, wherein the at least one seal comprises at least one O-ring disposed around the moveable member.
5. The hydraulically actuated valve device of clause 1 or of any of the clauses 1 to 4, wherein the valve closing member comprises a diaphragm located within the cavity of the valve body.
6. The hydraulically actuated valve device of clause 5, wherein the diaphragm is a deformable diaphragm configured to be in a first state when the fluid pressure of operating fluid in the inner cavity of the valve body is below the closing pressure, and to deform from the first state to a second state when the fluid pressure of the operating fluid in the inner cavity of the valve body is at or above the closing pressure, wherein the deformable diaphragm enables the first fluid to flow from the inlet channel to the outlet channel when the deformable diaphragm is in the first state, and wherein the deformable diaphragm obstructs at least one of the inlet channel and the outlet channel to inhibit flow of the first fluid from the inlet channel to the outlet channel when the deformable diaphragm is in the second state.
7. The hydraulically actuated valve device of clause 5 or 6, wherein the valve body has a valve seat in a flow path between the inlet channel and the outlet channel, and wherein the diaphragm is configured to enable flow of the first fluid from the inlet channel, through the flow path, to the outlet channel when the fluid pressure of the operating fluid in the inner cavity of the valve body is below the closing pressure, and to seal against the valve seat and inhibit flow of the first fluid to the outlet channel when the fluid pressure of the operating fluid in the inner cavity of the valve body is at or above the closing pressure.
8. The hydraulically actuated valve device of clause 7 or of any of the clauses 5 to 7, wherein the diaphragm is a deformable diaphragm configured to deform from a first state to a second state as the fluid pressure of the operating fluid in the inner cavity of the valve body is raised from below to above the closing pressure.
9. The hydraulically actuated valve device of clause 5 or of any of the clauses 5 to 8, further comprising a flexible tubing in the inner cavity of the valve body and extending between the inlet channel and the outlet channel, for flow of the first fluid from the inlet channel to the outlet channel when the fluid pressure of the operating fluid in the inner cavity of the valve body is below the closing pressure, wherein the diaphragm has a protruding obstruction feature configured to press against and close the flexible tubing to inhibit flow of the first fluid from the inlet channel to the outlet channel when the fluid pressure of the operating fluid in the inner cavity of the valve body is at or above the closing pressure.
10. The hydraulically actuated valve device of clause 5 or of any of the clauses 5 to 9, further including a needle guard comprising a rigid body resistant to being pierced by the needle, wherein the rigid body of the needle guard is located in the inner cavity of the valve body between the septum and the diaphragm, to protect the diaphragm from needle piercing.
11. The hydraulically actuated valve device of clause 10, wherein the rigid body of the needle guard includes at least one fluid flow passage through which the operating fluid may flow when the operating fluid is injected into the inner cavity of the valve body.
12. The hydraulically actuated valve device of clause 10 or 11, wherein the rigid body of the needle guard has an outer peripheral edge that includes a plurality channels defining fluid flow passages through which the operating fluid may flow when the operating fluid is injected into the inner cavity of the valve body.
13. The hydraulically actuated valve device of clause 1 or of any of the clauses 1 to 12, wherein the valve closing member comprises a flexible tubing in the inner cavity of the valve body and extending between the inlet channel and the outlet channel, for flow of the first fluid from the inlet channel to the outlet channel when the fluid pressure of the operating fluid in the inner cavity of the valve body is below the closing pressure, the flexible tubing configured to collapse under fluid pressure of the operating fluid in the inner cavity of the valve body and inhibit flow of the first fluid from the inlet channel to the outlet channel when the fluid pressure of the operating fluid in the inner cavity of the valve body is at or above the closing pressure.
14. The hydraulically actuated valve device of clause 13, further including a needle guard comprising a rigid body resistant to being pierced by the needle, wherein the rigid body of the needle guard is located in the inner cavity of the valve body between the septum and the flexible tubing of the valve closing member, to protect the flexible tubing from needle piercing.
15. The hydraulically actuated valve device of clause 14, wherein the rigid body of the needle guard includes at least one fluid flow passage through which the operating fluid may flow when the operating fluid is injected into the inner cavity of the valve body.
16. The hydraulically actuated valve device of clause 1 or of any of the clauses 1 to 15, further comprising a needle guide located on the open end of the valve body for guiding the needle toward a central portion of the septum.
17. The hydraulically actuated valve device of clause 16, wherein the needle guide comprises an annular body having a tapered surface extending to a central opening through which the needle may be passed.
18. An implantable medical device having the hydraulically actuated valve device of clause 1 or of any of the clauses 1 to 17 and a fluid outlet port connected to the outlet channel of the valve body.
19. An implantable medical device having the hydraulically actuated valve device of clause 1 or of any of the clauses 1 to 17 and a fluid inlet port connected to the inlet channel of the valve body.
20. An implantable medical device having an outlet port, an infusion media pump for selectively delivering the first fluid to the outlet port, and the hydraulically actuated valve device of clause 1 or of any of the clauses 1 to 17 connected between the infusion media pump and the outlet port.

## Claims

1. A hydraulically actuated valve device, comprising:
a valve body having an inner cavity, an inlet channel extending to the inner cavity, an outlet channel extending from the inner cavity, and an open end that opens to the inner cavity;
a septum located in the inner cavity, the septum being arranged to seal the open end of the valve body and being configured to receive a needle through which an operating fluid may be selectively injected into the inner cavity of the valve body to increase a fluid pressure in the inner cavity of the valve body, or selectively withdrawn from the inner cavity to reduce the fluid pressure in the inner cavity;
a valve closing member arranged in the inner cavity of the valve body, the valve closing member having an unactuated state to enable a first fluid to flow from the inlet channel to the outlet channel when the fluid pressure of operating fluid in the inner cavity of the valve body is below a closing pressure, the valve closing member having an actuated state that inhibits the first fluid from flowing into the inner cavity from the inlet channel or out of the inner cavity through the outlet channel when the operating fluid in the inner cavity of the valve body is at or above the closing pressure.

2. The hydraulically actuated valve device of claim 1, wherein the valve closing member comprises a moveable member that is configured to be in a first position within the inner cavity of the valve body when the fluid pressure of operating fluid in the inner cavity of the valve body is below the closing pressure, and to move to a second position within the inner cavity of the valve body when the fluid pressure of the operating fluid in the inner cavity of the valve body is at or above the closing pressure, wherein the moveable member enables the first fluid to flow from the inlet channel to the outlet channel when the moveable member is in the first position, and wherein the moveable member obstructs at least one of the inlet channel and the outlet channel to inhibit flow of the first fluid from the inlet channel to the outlet channel when the moveable member is in the second position.

3. The hydraulically actuated valve device of claim 2, wherein the moveable member includes at least one seal member that provides a fluid seal between the moveable member and an inner wall of the cavity of the valve body, particularly
wherein the at least one seal comprises at least one O-ring disposed around the moveable member.

4. The hydraulically actuated valve device of any of the claims 1 to 3, wherein the valve closing member comprises a diaphragm located within the cavity of the valve body.

5. The hydraulically actuated valve device of claim 4, wherein the diaphragm is a deformable diaphragm configured to be in a first state when the fluid pressure of operating fluid in the inner cavity of the valve body is below the closing pressure, and to deform from the first state to a second state when the fluid pressure of the operating fluid in the inner cavity of the valve body is at or above the closing pressure, wherein the deformable diaphragm enables the first fluid to flow from the inlet channel to the outlet channel when the deformable diaphragm is in the first state, and wherein the deformable diaphragm obstructs at least one of the inlet channel and the outlet channel to inhibit flow of the first fluid from the inlet channel to the outlet channel when the deformable diaphragm is in the second state.

6. The hydraulically actuated valve device of claim 4 or 5, wherein the valve body has a valve seat in a flow path between the inlet channel and the outlet channel, and wherein the diaphragm is configured to enable flow of the first fluid from the inlet channel, through the flow path, to the outlet channel when the fluid pressure of the operating fluid in the inner cavity of the valve body is below the closing pressure, and to seal against the valve seat and inhibit flow of the first fluid to the outlet channel when the fluid pressure of the operating fluid in the inner cavity of the valve body is at or above the closing pressure.

7. The hydraulically actuated valve device of any of the claims 4 to 6, wherein the diaphragm is a deformable diaphragm configured to deform from a first state to a second state as the fluid pressure of the operating fluid in the inner cavity of the valve body is raised from below to above the closing pressure.

8. The hydraulically actuated valve device of any of the claims 4 to 7, further comprising a flexible tubing in the inner cavity of the valve body and extending between the inlet channel and the outlet channel, for flow of the first fluid from the inlet channel to the outlet channel when the fluid pressure of the operating fluid in the inner cavity of the valve body is below the closing pressure, wherein the diaphragm has a protruding obstruction feature configured to press against and close the flexible tubing to inhibit flow of the first fluid from the inlet channel to the outlet channel when the fluid pressure of the operating fluid in the inner cavity of the valve body is at or above the closing pressure.

9. The hydraulically actuated valve device of any of the claims 4 to 8, further including a needle guard comprising a rigid body resistant to being pierced by the needle, wherein the rigid body of the needle guard is located in the inner cavity of the valve body between the septum and the diaphragm, to protect the diaphragm from needle piercing.

10. The hydraulically actuated valve device of claim 9, wherein the rigid body of the needle guard includes at least one fluid flow passage through which the operating fluid may flow when the operating fluid is injected into the inner cavity of the valve body, and/or
wherein the rigid body of the needle guard has an outer peripheral edge that includes a plurality channels defining fluid flow passages through which the operating fluid may flow when the operating fluid is injected into the inner cavity of the valve body.

11. The hydraulically actuated valve device of any of the claims 1 to 10, wherein the valve closing member comprises a flexible tubing in the inner cavity of the valve body and extending between the inlet channel and the outlet channel, for flow of the first fluid from the inlet channel to the outlet channel when the fluid pressure of the operating fluid in the inner cavity of the valve body is below the closing pressure, the flexible tubing configured to collapse under fluid pressure of the operating fluid in the inner cavity of the valve body and inhibit flow of the first fluid from the inlet channel to the outlet channel when the fluid pressure of the operating fluid in the inner cavity of the valve body is at or above the closing pressure, particularly
further including a needle guard comprising a rigid body resistant to being pierced by the needle, wherein the rigid body of the needle guard is located in the inner cavity of the valve body between the septum and the flexible tubing of the valve closing member, to protect the flexible tubing from needle piercing, particularly
wherein the rigid body of the needle guard includes at least one fluid flow passage through which the operating fluid may flow when the operating fluid is injected into the inner cavity of the valve body.

12. The hydraulically actuated valve device of any of the claims 1 to 11, further comprising a needle guide located on the open end of the valve body for guiding the needle toward a central portion of the septum, particularly
wherein the needle guide comprises an annular body having a tapered surface extending to a central opening through which the needle may be passed.

13. An implantable medical device having the hydraulically actuated valve device of any of the claims 1 to 12 and a fluid outlet port connected to the outlet channel of the valve body.

14. An implantable medical device having the hydraulically actuated valve device of any of the claims 1 to 12 and a fluid inlet port connected to the inlet channel of the valve body.

15. An implantable medical device having an outlet port, an infusion media pump for selectively delivering the first fluid to the outlet port, and the hydraulically actuated valve device of any of the claims 1 to 12 connected between the infusion media pump and the outlet port.
